# EUROPÄISCHE PATENTANMELDUNG

(11) **EP 0 690 048 A1**
(43) Veröffentlichungstag der Anmeldung: **03.01.1996**
(21) Anmeldenummer: 95109781.5
(22) Anmeldetag: 23.06.1995
(51) Int. Cl.: C07C 279/22, C07D 295/14, C07C 311/39, A61K 31/155, A61K 31/33

(54) **Ortho-amino-substituierte Benzoylguanidine, Verfahren zu ihrer Herstellung, ihre Verwendung als Medikament oder Diagnostikum sowie sie enthaltendes Medikament**

(30) Priorität: 29.06.1994 DE 4422685
(71) Anmelder: HOECHST AKTIENGESELLSCHAFT, D-65929 Frankfurt am Main (DE)
(72) Erfinder: Schwark, Jan-Robert, Dr., D-65929 Frankfurt (DE); Kleemann, Heinz-Werner, Dr., D-65474 Bischofsheim (DE); Lang, Hans-Jochen, Dr., D-65719 Hofheim (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(57) **Zusammenfassung**

Beschrieben werden ortho-amino-substituierte Benzoylguanidine der Formel I 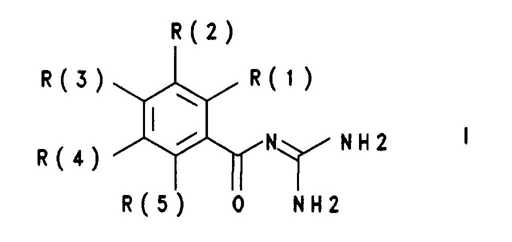
worin:
R(1) NR(50)R(6),
R(50) und R(6)
unabhängig voneinander Wasserstoff, (C₁-C₈)-Alkyl oder (C₁-C₈)-Perfluoralkyl bedeuten und
R(2), R(3), R(4) und R(5) die im Text angegebenen Bedeutungen haben. Sie werden erhalten durch Umsetzung von Verbindungen der Formel II 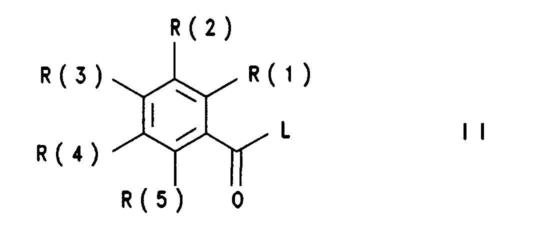
worin R(1) bis R(5) die oben angegebenen Bedeutungen haben und worin L für eine leicht nucleophil substituierbare Fluchtgruppe steht, mit Guanidin.

Verbindungen I sind hervorragend zur Behandlung von Erkrankungen des Herz-Kreislauf-Systems geeignet.

## Beschreibung

Die Erfindung betrifft ortho-amino-substituierte Benzoylguanidine der Formel I 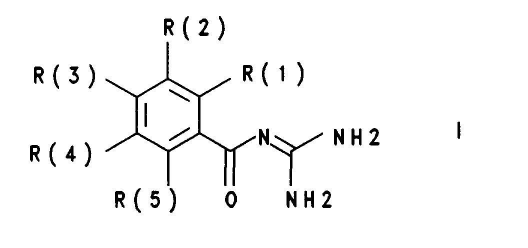
worin bedeuten:
- R(1): NR(50)R(6),
- R(50) und R(6): unabhängig voneinander Wasserstoff, (C₁-C₈)-Alkyl oder (C₁-C₈)-Perfluoralkyl;
- R(2), R(3), R(4) und R(5): unabhängig voneinander R(10)-SOₐ-, R(11)R(12)N-CO-, R(13)-CO- oder R(14)R(15)N-SO₂-;
- a: Null, 1 oder 2,
- R(10), R(11), R(12), R(13), R(14) und R(15): unabhängig voneinander (C₁-C₈)-Alkyl, (C₁-C₈)-Perfluoralkyl, (C₃-C₆)-Alkenyl oder -C_{ab}H_{2ab}-R(16);
- ab: Null, 1, 2, 3 oder 4;
- R(16): (C₃-C₇)-Cycloalkyl, Phenyl,
welches nicht substituiert ist oder substituiert mit 1 - 3 Substituenten ausgewählt aus der Gruppe bestehend aus F, Cl, CF₃, Methyl, Methoxy oder NR(17)R(18);
- R(17) und R(18): unabhängig voneinander H, CF₃ oder (C₁-C₄₎-Alkyl;
oder
- R(11), R(12), sowie R(14) und R(15): gemeinsam 4 oder 5 Methylengruppen, von denen eine CH₂-Gruppe durch Sauerstoff, S, NH, N-CH₃ oder N-Benzyl ersetzt sein kann;
oder
- R(11), R(12), R(14) und R(15): unabhängig voneinander Wasserstoff;
oder
- R(2), R(3), R(4) und R(5): unabhängig voneinander SR(21), -OR(22), -NR(23)R(24) oder -CR(25)R(26)R(27);
- R(21), R(22), R(23) und R(25): unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
- b: Null, 1 oder 2;
- R(24), R(26) und R(27): unabhängig voneinander Wasserstoff, (C₁-C₄)-Alkyl oder (C₁-C₄)-Perfluoralkyl;
oder
- R(2), R(3), R(4) und R(5): unabhängig voneinander Wasserstoff, F, Cl, Br, I, CN, -(Xa)_{dg}-C_{da}H_{2da+1}, -(Xb)_{dh}-(CH₂)_{db}-C_{de}F_{2de+1}, (C₃-C₈)-Alkenyl oder -C_{df}H_{2df}R(30);
- (Xa): O, S oder NR(33);
- R(33): H, (C₁-C₄)-Alkyl oder (C₁-C₄)-Perfluoralkyl;
- dg: Null oder 1;
- (Xb): O, S oder NR(34);
- R(34): H, (C₁-C₄)-Alkyl oder (C₁-C₄)-Perfluoralkyl;
- dh: Null oder 1;
- da: Null, 1, 2, 3, 4, 5, 6, 7, 8;
- db: Null, 1, 2, 3, 4;
- de: Null, 1, 2, 3, 4, 5, 6, 7;
- df: Null, 1, 2, 3, 4;
- R(30): (C₃-C₈)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
- R(31) und R(32): H, (C₁-C₄)-Alkyl oder (C₁-C₄)-Perfluoralkyl;
oder
- R(2), R(3), R(4) und R(5): unabhängig voneinander NR(40)R(41) oder -(Xe)-(CH₂)_{eb}R(45);
- R(40) und R(41): unabhängig voneinander Wasserstoff, (C₁-C₈)-Alkyl, (C₁-C₈)-Perfluoralkyl oder (CH₂)ₑ-R(42);
- e: Null, 1, 2, 3 oder 4;
- R(42): (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44);
- R(43) und R(44): unabhängig voneinander H, CF₃ oder (C₁-C₄)-Alkyl;
oder
- R(40) und R(41): gemeinsam 4 oder 5 Methylengruppen, von denen eine CH₂-Gruppe durch Sauerstoff, Schwefel, NH, N-CH₃ oder N-Benzyl ersetzt sein kann;
- (Xe): O, S oder NR(47);
- R(47): H, (C₁-C₄)-Alkyl oder (C₁-C₄)-Perfluoralkyl;
- eb: Null, 1, 2, 3 oder 4;
- R(45): (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
- Xfa: CH₂, O, S oder NR(48);
- Xfb: O, S oder NR(49);
- ed: 1, 2, 3, 4;
- R(46): H, (C₁-C₄)-Alkyl oder (C₁-C₄)-Perfluoralkyl;
- R(48), R(49), R(50) und R(51): unabhängig voneinander H oder (C₁-C₄)-Alkyl oder (C₁-C₄)-Perfluoralkyl;
wobei R(3) und R(4) jedoch nicht Wasserstoff sein können,
sowie deren pharmazeutisch verträgliche Salze.

Bevorzugt sind Verbindungen der Formel I, in der bedeuten:
- R(1): NR(50)R(6);
- R(50) und R(6): unabhängig voneinander Wasserstoff, CF₃ oder (C₁-C₄)-Alkyl;
- R(2) und R(5): Wasserstoff;
- R(4): R(10)-SOₐ-, R(11)R(12)N-CO-, R(13)-CO- oder R(14)R(15)N-SO₂-,
- a: Null, 1 oder 2;
- R(10), R(11), R(12), R(13), R(14) und R(15): unabhängig voneinander (C₁-C₈)-Alkyl, (C₁-C₈)-Perfluoralkyl, (C₃-C₆)-Alkenyl oder -C_{ab}H_{2ab}-R(16);
- ab: Null, 1, 2, 3 oder 4;
- R(16): (C₃-C₇)-Cycloalkyl oder Phenyl, welches nicht substituiert ist oder substituiert mit 1 - 3 Substituenten aus der Gruppe bestehend aus F, Cl, CF₃, Methyl, Methoxy oder NR(17)R(18);
- R(17) und R(18): unabhängig voneinander H, CF₃ oder (C₁-C₄₎-Alkyl;
oder
- R(11) und R(12), sowie R(14) und R(15): gemeinsam 4 oder 5 Methylengruppen, von denen eine CH₂-Gruppe durch Sauerstoff, S, NH, N-CH₃ oder N-Benzyl ersetzt sein kann;
oder
- R(11), R(12), R(14) und R(15): unabhängig voneinander auch Wasserstoff;
- R(3): SR(21), -OR(22), -NR(23)R(24) oder-CR(25)R(26)R(27);
- R(21), R(22), R(23) und R(25): unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
- b: Null, 1 oder 2;
- R(24), R(26) und R(27): unabhängig voneinander Wasserstoff, (C₁-C₄)-Alkyl oder (C₁-C₄)-Perfluoralkyl;
oder
- R(3) und R(4): unabhängig voneinander F, Cl, Br, I, CN, -(Xa)_{dg}-C_{da}H_{2da+1} oder -(Xb)_{dh}-(CH₂)_{db}-C_{de}F_{2de+1}, (C₃-C₈)-Alkenyl oder -C_{df}H_{2df}R(30);
- (Xa): O, S oder NR(33);
- R(33): H, (C₁-C₄)-Alkyl oder (C₁-C₄)-Perfluoralkyl;
- dg: Null, 1;
- (Xb): O, S oder NR(34);
- R(34): H, (C₁-C₄)-Alkyl oder (C₁-C₄)-Perfluoralkyl;
- dh: Null oder 1;
- da: Null, 1, 2, 3, 4, 5, 6, 7 oder 8;
- db: Null, 1, 2, 3 oder 4;
- de: Null, 1, 2, 3, 4, 5, 6 oder 7;
- df: Null, 1, 2, 3 oder 4;
- R(30): (C₃-C₈)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
- R(31) und R(32): unabhängig voneinander H, (C₁-C₄)-Alkyl oder (C₁-C₄)-Perfluoralkyl;
oder
- R(3): NR(40)R(41) oder (Xe)-(CH₂)_{eb}R(45),
- R(40) und R(41): unabhängig voneinander Wasserstoff, (C₁-C₈)-Alkyl, (C₁-C₈)-Perfluoralkyl oder (CH₂)ₑ-R(42);
- e: Null, 1, 2, 3 oder 4;
- R(42): (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44);
- R(43) und R(44): unabhängig voneinander H, CF₃ oder C₁-C₄-Alkyl;
oder
- R(40) und R(41): gemeinsam 4 oder 5 Methylengruppen, von denen eine CH₂-Gruppe durch Sauerstoff, Schwefel, NH, N-CH₃ oder N-Benzyl ersetzt sein kann;
- (Xe): O, S oder NR(47);
- R(47): H, (C₁-C₄)-Alkyl oder (C₁-C₄)-Perfluoralkyl;
- eb: Null, 1, 2, 3 oder 4;
- R(45): (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
- (Xfa): CH₂, O, S oder NR(48);
- (Xfb): O, S oder NR(49);
- ed: 1, 2, 3 oder 4;
- R(46): H, (C₁-C₄)-Alkyl oder (C₁-C₄)-Perfluoralkyl;
- R(48), R(49), R(50) und R(51): unabhängig voneinander H, (C₁-C₄)-Alkyl oder (C₁-C₄)-Perfluoralkyl;
sowie deren pharmazeutisch verträgliche Salze.

Besonders bevorzugt sind Verbindungen der Formel I, in welcher bedeuten:
- R(1): NR(50)R(6),
- R(50) und R(6): unabhängig voneinander Wasserstoff oder CH₃;
- R(2) und R(5): Wasserstoff;
- R(4): R(10)-SOₐ-, R(11)R(12)N-CO-, R(13)-CO- oder R(14)R(15)N-SO₂-;
- a: 2;
- R(10), R(11), R(12), R(13), R(14) und R(15): unabhängig voneinander (C₁-C₈)-Alkyl, (C₁-C₈)-Perfluoralkyl oder -C_{ab}H_{2ab}-R(16);
- ab: Null, 1 oder 2;
- R(16): Phenyl, welches nicht substituiert ist oder substituiert mit 1 - 3 Substituenten ausgewählt aus der Gruppe bestehend aus F, Cl, CF₃, Methyl, Methoxy und NR(17)R(18);
- R(17) und R(18): unabhängig voneinander H, CF₃ oder CH₃;
oder
- R(11) und R(12), sowie R(14) und R(15): gemeinsam 4 oder 5 Methylengruppen, von denen eine CH₂-Gruppe durch Sauerstoff, S, NH, N-CH₃ oder N-Benzyl ersetzt sein kann;
oder
- R(11), R(12), R(14) und R(15): unabhängig voneinander Wasserstoff;
- R(3): -OR(22) oder -NR(23)R(24);
- R(22) und R(23): unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
- b: Null, 1 oder 2;
- R(24): Wasserstoff, CH₃ oder CF₃;
oder
- R(3) und R(4): unabhängig voneinander F, Cl, Br, I, CN, -(Xa)_{dg}-C_{da}H_{2d+1} oder -(Xb)_{dh}-(CH₂)_{db}-C_{de}F_{2de+1};
- (Xa): O, S oder NR(33);
- R(33): H, (C₁-C₄)-Alkyl oder (C₁-C₄)-Perfluoralkyl;
- dg: Null;
- (Xb): O, S oder NR(34);
- R(34): H, (C₁-C₄)-Alkyl oder (C₁-C₄)-Perfluoralkyl;
- dh: Null;
- da: 1, 2, 3, 4;
- db: Null;
- de: 1, 2, 3, 4;
oder
- R(3): NR(40)R(41) oder (Xe)-(CH₂)_{eb}R(45);
- R(40) und R(41): unabhängig voneinander Wasserstoff, (C₁-C₈)-Alkyl, (C₁-C₈)-Perfluoralkyl oder (CH₂)ₑ-R(42);
- e: Null, 1 oder 2;
- R(42): (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44);
- R(43) und R(44): unabhängig voneinander H, CF₃ oder CH₃;
oder
- R(40) und R(41): gemeinsam 4 oder 5 Methylengruppen, von denen eine CH₂-Gruppe durch Sauerstoff, Schwefel, NH, N-CH₃ oder N-Benzyl ersetzt sein kann;
- (Xe): O, S oder NR(47);
- R(47): H, (C₁-C₄)-Alkyl oder CF₃;
- eb: Null, 1 oder 2;
- R(45): (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
- (Xfa): CH₂, O, S oder NR(48);
- (Xfb): O, S, NR(49);
- ed: 1 oder 2;
- R(46): H, (C₁-C₄)-Alkyl oder CF₃;
- R(48), R(49), R(50) und R(51): unabhängig voneinander H, (C₁-C₄)-Alkyl oder CF₃;
sowie deren pharmazeutisch verträgliche Salze.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(5) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkyl- und Perfluoralkylreste können sowohl geradkettig wie verzweigt vorliegen.

Die Erfindung betrifft weiterhin ein Verfahren zur Herstellung der Verbindungen I, dadurch gekennzeichnet,
daß man Verbindungen der Formel II 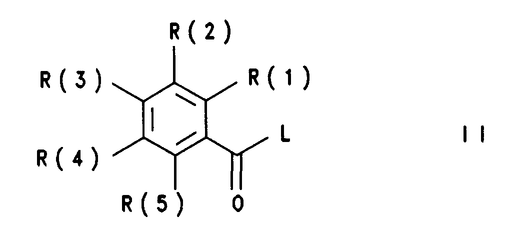
worin R(1) bis R(5) die oben angegebenen Bedeutungen haben und worin L für eine leicht nucleophil substituierbare Fluchtgruppe steht,
mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann. Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1,351-367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)-methylen)amino]-1,1,3,3-tetramethyluronium-tetrafluoroborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF zwischen 20°C und Siedetemperatur dieser Lösungsmittel bewährt. Bei den meisten Umsetzungen von Verbindungen II mit salzfreiem Guanidin wurde vorteilhaft in inerten Lösungsmitteln wie THF, Dimethoxyethan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigem Guanidin zur Abbindung der Halogenwasserstoffsäure.

Die Einführung von substituierten Schwefel-, Sauerstoff- oder Stickstoffnucleophilen gelingt durch literaturbekannte Methoden der nucleophilen Substitution am Aromaten. Als Abgangsgruppe haben sich bei dieser Substitution Halogenide und Trifluormethansulfonate bewährt. Man arbeitet vorteilhaft in einem dipolar aprotischen Lösungsmittel, wie zum Beispiel DMF oder TMU bei einer Temperatur zwischen 0°C und dem Siedepunkt des Lösungsmittels, bevorzugt zwischen 80°C und dem Siedepunkt des Lösungsmittels. Als Säurefänger dient vorteilhaft ein Alkali- oder Erdalkalisalz mit einem Anion hoher Basizität und geringer Nucleophilie, wie zum Beispiel K₂CO₃.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 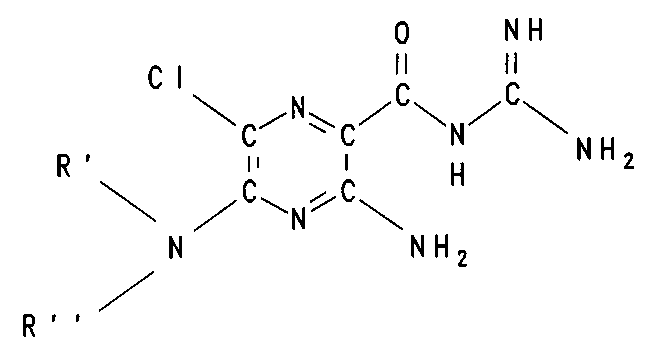
Amilorid: R',R'' = H
Dimethylamilorid: R',R'' = CH₃
Ethylisopropylamilorid: R' = C₂H₅, R'' = CH(CH₃)₂
Darüberhinaus sind Untersuchungen bekannt geworden, die auf antiarrhythmische Eigenschaften von Amilorid hinweisen [Circulation 79, 1257 - 63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988), book of abstracts]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Es war überraschend, daß die erfindungsgemäßen Verbindungen keine unerwünschten und nachteiligen saliduretischen, jedoch sehr gute antiarrhythmische Eigenschaften aufweisen; sie sind deshalb gut geeignet zum Behandeln von Zuständen, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten und chronischen durch Ischämie ausgelösten Schäden oder dadurch primär oder sekundär induzierten Krankheiten verwendet werden. Dies betrifft ihre Verwendung als Arzneimittel für operative Eingriffe, z. B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielsweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielsweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist.

Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis 10 mg, vorzugsweise 1 mg. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

Analog der in den Ausführungsbeispielen angegebenen Vorschriften können die nachfolgend aufgeführten erfindungsgemäßen Verbindungen der Formel I bzw. deren physiologisch verträglichen Salze hergestellt werden:

### Liste der Abkürzungen:

- MeOH: Methanol
- DMF: N,N-Dimethylformamid
- TMU: N,N,N',N'-Tetramethylharnstoff
- NBS: N-Bromsuccinimid
- AIBN: α,α-Azo-bis-isobytyronitril
- EI: electron impact
- DCI: Desorption-Chemical Ionisation
- RT: Raumtemperatur
- EE: Ethylacetat
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-Ionisation
- Me: Methyl
- Et: Ethyl
- Bn: Benzyl
- ZNS: Zentralnervensystem
- Brine: gesättigte wäßrige NaCl-Lösung

### Experimenteller Teil

### Allgemeine Vorschrift zur Herstellung von Acyl-guanidinen (I)

### Variante A: aus Carbonsäuren (II, L = OH)

1.0 eq. des Carbonsäurederivates der Formel II löst bzw. suspendiert man in wasserfreiem THF (5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotavapor) ab, versetzt mit Wasser, stellt mit 2N HCl auf pH 6 bis 7 und filtriert das entsprechende Acylguanidin (Formel I) ab. Die so erhaltenen Acyl-guanidine können durch Behandeln mit wäßriger, methanolischer oder etherischer Salzsäure oder anderen pharmakologisch verträglichen Säuren in die entsprechenden Salze übergeführt werden.

Allgemeine Vorschrift zur Herstellung von Acyl-guanidinen (I)
Variante B: aus Carbonsäure-alkylestern (II, L = O-Alkyl)
1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotavapor) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1

2-Amino-4-chloro-5-sulfamoyl-benzoylguanidin-dihydrochlorid
wurde gemäß Variante B aus dem entsprechenden Benzoesäureester dargestellt.
mp: 288 - 290°C

### Beispiel 2

2-Amino-4-chloro-5-(N-ethyl-sulfamoyl)-benzoyl-guanidin-dihydrochlorid
Wurde gemäß Variante B aus dem entsprechenden Benzoesäureester dargestellt.
mp: 242°C

### Beispiel 3

2-(N-Butyl-amino)-4-chloro-5-sulfamoyl-benzoylguanidin-dihydrochlorid
wurde gemäß Variante B aus dem entsprechenden Benzoesäureester dargestellt.
mp: 286°C

### Beispiel 4

2-(N-Ethyl-amino)-4-chloro-5-sulfamoyl-benzoylguanidin-dihydrochlorid
wurde gemäß Variante B aus dem entsprechenden Benzoesäureester dargestellt.
mp: 290°C

### Beispiel 5

2-Amino-5-(1-piperidyl)-benzoylguanidin-dihydrochlorid
wurde gemäß Variante B aus dem entsprechenden Benzoesäureester dargestellt.
mp: 170°C

### Beispiel 6

2-Amino-3-bromo-5-methyl-benzoylguanidin-dihydrochlorid
wurde gemäß Variante A aus der entsprechenden Benzoesäure dargestellt.
mp: > 300°C

### Beispiel 7

2-Amino-3-methyl-benzoylguanidin-dihydrochlorid
wurde analog Variante B aus dem entsprechenden Benzoesäuremethylester dargestellt.
mp: > 270°C

### Beispiel 8

2-Amino-3,5-dichloro-benzoylguanidin-dihydrochlorid
wurde gemäß Variante A aus der entsprechenden Bnezoesäure dargestellt.
mp: 190°C

### Beispiel 9

2-Amino-5-chloro-benzoylguanidin-dihydrochlorid
wurde gemäß Variante A aus der entsprechenden Benzoesäure dargestellt.
mp: > 390°C unter Zersetzung
R_{F} = 0.27 (Ethylacetat)

### Beispiel 10

2-Amino-4,5-dimethoxy-benzoylguanidin-dihydrochlorid
wurde gemäß Variante A aus der entsprechenden Benzoesäure dargestellt.
mp: 177°C

### Beispiel 11

2-Amino-4,5-dibromo-benzoylguanidin
wurde gemäß Variante B aus dem entsprechenden Benzoesäuremethylester dargestellt.
mp: 158°C

### Beispiel 12

2-Dimethylamino-5-chloro-benzoylguanidin-dihydrochlorid
wurde gemäß Variante B aus dem entsprechenden Benzoesäuremethylester dargestellt.
mp: 135 - 145°C

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

### Inhibition des Na⁺/H⁺-Exchangers:

| Beispiel | IC₅₀ [µmol/l] |
|---|---|
| 1 | 2 - 3 |
| 9 | 1 - 2 |
| 11 | 0.1 |
| 12 | 1 - 2 |

## Patentansprüche

1. Ortho-amino-substituierte Benzoylguanidine der Formel I 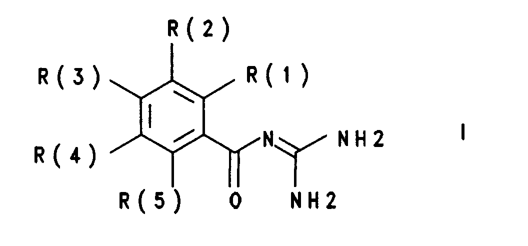 worin bedeuten:
R(1) NR(50)R(6),
R(50) und R(6) unabhängig voneinander Wasserstoff, (C₁-C₈)-Alkyl oder (C₁-C₈)-Perfluoralkyl;
R(2), R(3), R(4) und R(5) unabhängig voneinander R(10)-SOₐ-, R(11)R(12)N-CO-, R(13)-CO- oder R(14)R(15)N-SO₂-;
a Null, 1 oder 2,
R(10), R(11), R(12), R(13), R(14) und R(15) unabhängig voneinander (C₁-C₈)-Alkyl, (C₁-C₈)-Perfluoralkyl, (C₃-C₆)-Alkenyl oder -C_{ab}H_{2ab}-R(16);
ab Null, 1, 2, 3 oder 4;
R(16) (C₃-C₇)-Cycloalkyl, Phenyl,
welches nicht substituiert ist oder substituiert mit 1 - 3 Substituenten ausgewählt aus der Gruppe bestehend aus F, Cl, CF₃, Methyl, Methoxy oder NR(17)R(18);
R(17) und R(18) unabhängig voneinander H, CF₃ oder (C₁-C₄₎-Alkyl;
oder
R(11), R(12), sowie R(14) und R(15) gemeinsam 4 oder 5 Methylengruppen, von denen eine CH₂-Gruppe durch Sauerstoff, S, NH, N-CH₃ oder N-Benzyl ersetzt sein kann;
oder
R(11), R(12), R(14) und R(15) unabhängig voneinander Wasserstoff;
oder
R(2), R(3), R(4) und R(5) unabhängig voneinander SR(21), -OR(22), -NR(23)R(24) oder -CR(25)R(26)R(27);
R(21), R(22), R(23) und R(25) unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
b Null, 1 oder 2;
R(24), R(26) und R(27) unabhängig voneinander Wasserstoff, (C₁-C₄)-Alkyl oder (C₁-C₄)-Perfluoralkyl;
oder
R(2), R(3), R(4) und R(5) unabhängig voneinander Wasserstoff, F, Cl, Br, I, CN, -(Xa)_{dg}-C_{da}H_{2da+1}, -(Xb)_{dh}-(CH₂)_{db}-C_{de}F_{2de+1}, (C₃-C₈)-Alkenyl oder -C_{df}H_{2df}R(30);
(Xa) O, S oder NR(33);
R(33) H, (C₁-C₄)-Alkyl oder (C₁-C₄)-Perfluoralkyl;
dg Null oder 1;
(Xb) O, S oder NR(34);
R(34) H, (C₁-C₄)-Alkyl oder (C₁-C₄)-Perfluoralkyl;
dh Null oder 1;
da Null, 1, 2, 3, 4, 5, 6, 7, 8;
db Null, 1, 2, 3, 4;
de Null, 1, 2, 3, 4, 5, 6, 7;
df Null, 1, 2, 3, 4;
R(30) (C₃-C₈)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
R(31) und R(32) H, (C₁-C₄)-Alkyl oder (C₁-C₄)-Perfluoralkyl;
oder
R(2), R(3), R(4) und R(5) unabhängig voneinander NR(40)R(41) oder -(Xe)-(CH₂)_{eb}R(45);
R(40) und R(41) unabhängig voneinander Wasserstoff, (C₁-C₈)-Alkyl, (C₁-C₈)-Perfluoralkyl oder (CH₂)ₑ-R(42);
e Null, 1, 2, 3 oder 4;
R(42) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44);
R(43) und R(44) unabhängig voneinander H, CF₃ oder (C₁-C₄)-Alkyl;
oder
R(40) und R(41) gemeinsam 4 oder 5 Methylengruppen, von denen eine CH₂-Gruppe durch Sauerstoff, Schwefel, NH, N-CH₃ oder N-Benzyl ersetzt sein kann;
(Xe) O, S oder NR(47);
R(47) H, (C₁-C₄)-Alkyl oder (C₁-C₄)-Perfluoralkyl;
eb Null, 1, 2, 3 oder 4;
R(45) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
Xfa CH₂, O, S oder NR(48);
Xfb O, S oder NR(49);
ed 1, 2, 3, 4;
R(46) H, (C₁-C₄)-Alkyl oder (C₁-C₄)-Perfluoralkyl;
R(48), R(49), R(50) und R(51) unabhängig voneinander H oder (C₁-C₄)-Alkyl oder (C₁-C₄)-Perfluoralkyl;
wobei R(3) und R(4) jedoch nicht Wasserstoff sein können,
sowie deren pharmazeutisch verträgliche Salze.

2. Verbindung der Formel I nach Anspruch 1, dadurch gekennzeichnet, daß darin bedeuten:
R(1) NR(50)R(6);
R(50) und R(6) unabhängig voneinander Wasserstoff, CF₃ oder (C₁-C₄)-Alkyl;
R(2) und R(5) Wasserstoff;
R(4) R(10)-SOₐ-, R(11)R(12)N-CO-, R(13)-CO- oder R(14)R(15)N-SO₂-;
a Null, 1 oder 2;
R(10), R(11), R(12), R(13), R(14) und R(15) unabhängig voneinander (C₁-C₈)-Alkyl, (C₁-C₈)-Perfluoralkyl, (C₃-C₆)-Alkenyl oder -C_{ab}H_{2ab}-R(16);
ab Null, 1, 2, 3 oder 4;
R(16) (C₃-C₇)-Cycloalkyl oder Phenyl, welches nicht substituiert ist oder substituiert mit 1 - 3 Substituenten aus der Gruppe bestehend aus F, Cl, CF₃, Methyl, Methoxy oder NR(17)R(18);
R(17) und R(18) unabhängig voneinander H, CF₃ oder (C₁-C₄)-Alkyl;
oder
R(11) und R(12), sowie R(14) und R(15) gemeinsam 4 oder 5 Methylengruppen, von denen eine CH₂-Gruppe durch Sauerstoff, S, NH, N-CH₃ oder N-Benzyl ersetzt sein kann;
oder
R(11), R(12), R(14) und R(15) unabhängig voneinander auch Wasserstoff;
R(3) SR(21), -OR(22), -NR(23)R(24) oder-CR(25)R(26)R(27);
R(21), R(22), R(23) und R(25) unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
b Null, 1 oder 2;
R(24), R(26) und R(27) unabhängig voneinander Wasserstoff, (C₁-C₄)-Alkyl oder (C₁-C₄)-Perfluoralkyl;
oder
R(3) und R(4) unabhängig voneinander F, Cl, Br, I, CN, -(Xa)_{dg}-C_{da}H_{2da+1} oder -(Xb)_{dh}-(CH₂)_{db}-C_{de}F_{2de+1}, (C₃-C₈)-Alkenyl oder -C_{df}H_{2df}R(30);
(Xa) O, S oder NR(33);
R(33) H, (C₁-C₄)-Alkyl oder (C₁-C₄)-Perfluoralkyl;
dg Null, 1;
(Xb) O, S oder NR(34);
R(34) H, (C₁-C₄)-Alkyl oder (C₁-C₄)-Perfluoralkyl;
dh Null oder 1;
da Null, 1, 2, 3, 4, 5, 6, 7 oder 8;
db Null, 1, 2, 3 oder 4;
de Null, 1, 2, 3, 4, 5, 6 oder 7;
df Null, 1, 2, 3 oder 4;
R(30) (C₃-C₈)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(31)R(32);
R(31) und R(32) unabhängig voneinander H, (C₁-C₄)-Alkyl oder (C₁-C₄)-Perfluoralkyl;
oder
R(3) NR(40)R(41) oder (Xe)-(CH₂)_{eb}R(45),
R(40) und R(41) unabhängig voneinander Wasserstoff, (C₁-C₈)-Alkyl, (C₁-C₈)-Perfluoralkyl oder (CH₂)ₑ-R(42);
e Null, 1, 2, 3 oder 4;
R(42) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44);
R(43) und R(44) unabhängig voneinander H, CF₃ oder C₁-C₄-Alkyl;
oder
R(40) und R(41) gemeinsam 4 oder 5 Methylengruppen, von denen eine CH₂-Gruppe durch Sauerstoff, Schwefel, NH, N-CH₃ oder N-Benzyl ersetzt sein kann;
(Xe) O, S oder NR(47);
R(47) H, (C₁-C₄)-Alkyl oder (C₁-C₄)-Perfluoralkyl;
eb Null, 1, 2, 3 oder 4;
R(45) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
(Xfa) CH₂, O, S oder NR(48);
(Xfb) O, S oder NR(49);
ed 1, 2, 3 oder 4;
R(46) H, (C₁-C₄)-Alkyl oder (C₁-C₄)-Perfluoralkyl;
R(48), R(49), R(50) und R(51) unabhängig voneinander H, (C₁-C₄)-Alkyl oder (C₁-C₄)-Perfluoralkyl.

3. Verbindung der Formel I nach mindestens einem der Ansprüche 1 bis 2, dadurch gekennzeichnet, daß darin bedeuten:
R(1) NR(50)R(6),
R(50) und R(6) unabhängig voneinander Wasserstoff oder CH₃;
R(2) und R(5) Wasserstoff;
R(4) R(10)-SOₐ-, R(11)R(12)N-CO-, R(13)-CO- oder R(14)R(15)N-SO₂-;
a 2;
R(10), R(11), R(12), R(13), R(14) und R(15) unabhängig voneinander (C₁-C₈)-Alkyl, (C₁-C₈)-Perfluoralkyl oder -C_{ab}H_{2ab}-R(16);
ab Null, 1 oder 2;
R(16) Phenyl, welches nicht substituiert ist oder substituiert mit 1 - 3 Substituenten ausgewählt aus der Gruppe bestehend aus F, Cl, CF₃, Methyl, Methoxy und NR(17)R(18);
R(17) und R(18) unabhängig voneinander H, CF₃ oder CH₃;
oder
R(11) und R(12), sowie R(14) und R(15) gemeinsam 4 oder 5 Methylengruppen, von denen eine CH₂-Gruppe durch Sauerstoff, S, NH, N-CH₃ oder N-Benzyl ersetzt sein kann;
oder
R(11), R(12), R(14) und R(15) unabhängig voneinander Wasserstoff;
R(3) -OR(22) oder -NR(23)R(24);
R(22) und R(23) unabhängig voneinander -C_{b}H_{2b}-(C₁-C₉)-Heteroaryl, das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
b Null, 1 oder 2;
R(24) Wasserstoff, CH₃ oder CF₃;
oder
R(3) und R(4) unabhängig voneinander F, Cl, Br, I, CN, -(Xa)_{dg}-C_{da}H_{2da+1} oder -(Xb)_{dh}-(CH₂)_{db}-C_{de}F_{2de+1};
(Xa) O, S oder NR(33);
R(33) H, (C₁-C₄)-Alkyl oder (C₁-C₄)-Perfluoralkyl;
dg Null;
(Xb) O, S oder NR(34);
R(34) H, (C₁-C₄)-Alkyl oder (C₁-C₄)-Perfluoralkyl;
dh Null;
da 1, 2, 3, 4;
db Null;
de 1, 2, 3, 4; oder
R(3) NR(40)R(41) oder (Xe)-(CH₂)_{eb}R(45);
R(40) und R(41) unabhängig voneinander Wasserstoff, (C₁-C₈)-Alkyl, (C₁-C₈)-Perfluoralkyl oder (CH₂)ₑ-R(42);
e Null, 1 oder 2;
R(42) (C₃-C₇)-Cycloalkyl, Phenyl, welches nicht substituiert ist oder substituiert mit 1 - 3 Substituenten ausgewählt aus der Gruppe bestehend aus F, Cl, CF₃, Methyl, Methoxy und NR(43)R(44);
R(43) und R(44) unabhängig voneinander H, CF₃ oder CH₃;
oder
R(40) und R(41) gemeinsam 4 oder 5 Methylengruppen, von denen eine CH₂-Gruppe durch Sauerstoff, Schwefel, NH, N-CH₃ oder N-Benzyl ersetzt sein kann;
(Xe) O, S oder NR(47);
R(47) H, (C₁-C₄)-Alkyl oder CF₃;
eb Null, 1 oder 2;
R(45) (C₃-C₇)-Cycloalkyl oder Phenyl, welches nicht substituiert ist oder substituiert mit 1 - 3 Substituenten ausgewählt aus der Gruppe bestehend aus F, Cl, CF₃, Methyl, Methoxy, NR(50)R(51) und -(Xfa)-(CH₂)_{ed}-(Xfb)R(46);
(Xfa) CH₂, O, S oder NR(48);
(Xfb) O, S, NR(49);
ed 1 oder 2;
R(46) H, (C₁-C₄)-Alkyl oder CF₃;
R(48), R(49), R(50) und R(51) unabhängig voneinander H, (C₁-C₄)-Alkyl oder CF₃.

4. Verfahren zum Herstellen einer Verbindung I nach Anspruch 1, dadurch gekennzeichnet, daß man eine Verbindung der Formel II 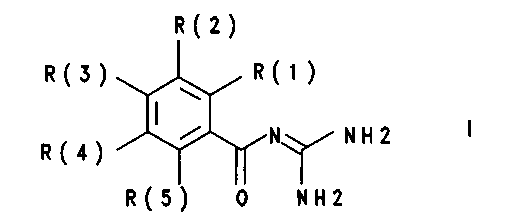 worin R(1) bis R(5) wie in Anspruch 1 definiert sind und worin L für eine leicht nucleophil substituierbare Fluchtgruppe steht
mit Guanidin umsetzt,
und daß man gegebenenfalls in ein pharmazeutisch verträgliches Salz überführt.

5. Verwendung einer Verbindung I nach Anspruch 1 zur Herstellung eines Medikaments zu Behandlung von Arrhythmien.

6. Methode zum Behandeln von Arrhythmien, dadurch gekennzeichnet, daß man eine wirksame Menge einer Verbindung I nach Anspruch 1 mit den üblichen Zusatzstoffen versetzt und in einer geeigneten Darreichungsform verabreicht.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen diabetische Spätkomplikationen, Krebserkrankungen, fibrotische Erkrankungen wie Lungenfibrose, Leberfibrose oder Nierenfibrose, Prostatahyperplasie.

16. Verwendung einer Verbindung I nach Anspruch 1 zur Herstellung eines wissenschaftliches Tools zur Inhibition des Na⁺/H⁺-Exchangers, zur Diagnose der Hypertonie und proliferativer Erkrankungen.

17. Heilmittel, enthaltend eine wirksame Menge einer Verbindung I nach einem oder mehreren der Ansprüche 1 bis 3.
